# EUROPEAN PATENT APPLICATION

(11) **EP 2 636 729 A1**
(43) Date of publication of application: **11.09.2013**
(21) Application number: 12158319.9
(22) Date of filing: 06.03.2012
(51) Int. Cl.: C12M 3/06

(54) **Cell culture device for microwell plate and fluidics system**

(71) Applicant: CSEM Centre Suisse d'Electronique et de Microtechnique SA - Recherche et Développement, 2002 Neuchâtel (CH)
(72) Inventor: Sbrana, Tommaso, 55054 Massarosa (IT); Ahluwalia, Arti, 54035 Fosdinovo (IT); Meister, André, 2314 La Sagne (CH); Favre, Mélanie, 2000 Neuchâtel (CH)
(74) Representative: GLN SA

(57) **Abstract**

The invention relates to a cell culture device comprising:
- a fluidics interface (30) comprising a lower (32) and an upper (34) compartments, each compartment comprising fluidics inlet (38) and outlet (40),
- a membrane holder (12) formed by a lower (16) and an upper (18) parts, said lower and upper parts being arranged to be fastened to each other and to retain a membrane suited to cell culture, each of said lower and upper parts comprising a first and a second openings allowing possible membrane permeation.

Said membrane holder (12) is sized and shaped to be introduced in and to cooperate with a microplate well. Said membrane holder (12) is sized and shaped to be lodged between the lower and upper compartments of said fluidics interface, in such a manner that said lower and upper compartments form, respectively with said first and second openings, a first and a second fluidics reaction chambers on each side of the membrane, intended to be connected with a fluidics system by said fluidics inlet (38) and outlet (40).

## Description

### Technical field

The present invention relates to the field of cell biology and cell culture. It more particularly relates to a cell culture device adapted to be used both with a microwell plate and with a fluidics system.

### State of the art

In vitro models of biological barriers (such as lung, skin, the intestines and the blood-brain barrier) are used, for example, to screen potential pharmaceuticals and toxins for their ability to enter into and to move around the human body. In vitro models consist of a single layer or multiple layers of cells that are cultured in the laboratory so as to mimic the properties of biological barriers in the body.

The function of biological barriers in the body is to divide the inside of the body from the outside (e.g skin, lungs, the intestines) or different compartments of the body from each other (e.g. the blood-brain barrier, the walls of blood vessels). In order to model this function in the laboratory, in vitro models of biological barriers are cultured on porous membranes. This is commonly done using microporous well inserts.

The well insert is used in combination with a microwell plate consisting of a number of wells made in plastic. The insert is inserted into a well, which it divides in two, a top (apical) compartment and a bottom (basolateral) compartment which communicate via a porous membrane at the bottom of the insert. Cells are added to the apical side of the well insert and are cultured on the porous membrane. Typically, the cells will grow to form a watertight layer that divides the apical from the basolateral compartment, as in the body.

To study, for example, pharmaceutical drug transport or permeation across the model biological barrier, candidate drugs are added to the apical compartment, which represents the outside of the body for a skin model, or the inside of the lungs, or of the intestines, etc.... Permeation is quantified by measuring the concentration of the candidate drug in the basolateral compartment, which represents the inside of the body, after a fixed time. These operations are carried out by pipetting into and from the different compartments of the microwell/insert combination. Pipetting may either be done by hand or using a pipetting robot.

A number of manufacturers produce porous microwell inserts for cell culture, including Millipore, Nunc, Corning, GreinerBioOne and BD Falcon.

A number of publications in the scientific literature have described the use of microporous membranes for cell culture in fluidics or microfluidics systems. For example, Huh et al. Science, 328, 1662 (2010), Hediger et al Biosensors & Bioelectronics 16 (2001) 689. In these systems, the cells are grown on the microporous membrane within the fluidics system and reagents and cell culture medium are transported to and from the cells by flow of liquid within the system.

The use of a fluidics system presents a number of advantages when compared to the use of a microwell plate and inserts. Firstly, it is easy to automate fluid handling: the systems used may be cheaper and more compact compared to pipetting robots. Secondly, liquid flow may more closely resemble the physiological condition of the biological barrier being studied e.g. the intestine or the walls of blood vessels. This will result in a model that is more physiologically relevant and that can be expected to give results that are closer to those observed in the body. Thirdly, it may be possible to work with smaller liquid volumes, thus reducing the costs associated with tests.

One disadvantage of the use of fluidics systems can be observed when they are used with cells that require a long culture time before they can be used as model biological barriers. This kind of behavior can be observed, for example, with the CaCo-2 C2BBe1 clone, a cell line derived from a colon cancer. Caco-2 cells must be cultured for 21 days before they differentiate to the intestinal phenotype, form tight junctions and can be used as a model of the intestine. During this long period of culture before the cells can be used as model biological barriers very few fluid handling/pipetting operations are necessary. Thus the presence of the cells in the fluidics system may necessitate the use of expensive pumps and bioreactors, which might more usefully be employed for experiments on mature cell layers.

A similar problem may be observed in complex fluidics systems in which more than one cell type is present for an experiment. It may be the case that the different cell types may take different times to reach maturity for the experiment. It is also likely that the different cell types grow best in different cell culture media, so that it may be difficult to culture all the different cell types to maturity in the same fluidics system.

Thus, the present invention aims to alleviate these problems and allow to use a culture substrate both with a microwell plate and with a fluidics system

### Summary of the invention

To this end, the invention concerns a cell culture device comprising:
- a fluidics interface comprising a lower and an upper compartments, each compartment comprising fluidics inlet and outlet,
- a membrane holder formed by a lower and an upper parts, said lower and upper parts being arranged to be fastened to each other and to retain a membrane suited to cell culture, each of said lower and upper parts comprising a first and a second openings allowing possible membrane permeation.

The membrane holder is sized and shaped to be introduced in and to cooperate with a microplate well. Moreover, the membrane holder is sized and shaped to be lodged between the lower and upper compartments of said fluidics interface, in such a manner that said lower and upper compartments form, respectively with said first and second openings, a first and a second fluidics reaction chambers on each side of the membrane, intended to be connected with a fluidics system by said fluidics inlet and outlet.

The invention also concerns a method for using a membrane with a device as mentioned above.

### Brief description of the figures

The accompanying drawings, which are incorporated in and constitute a part of this specification, illustrate embodiments of the invention, and, together with the general description of the invention given above, and the detailed description of the embodiments given below, serve to explain the principles of the present invention.

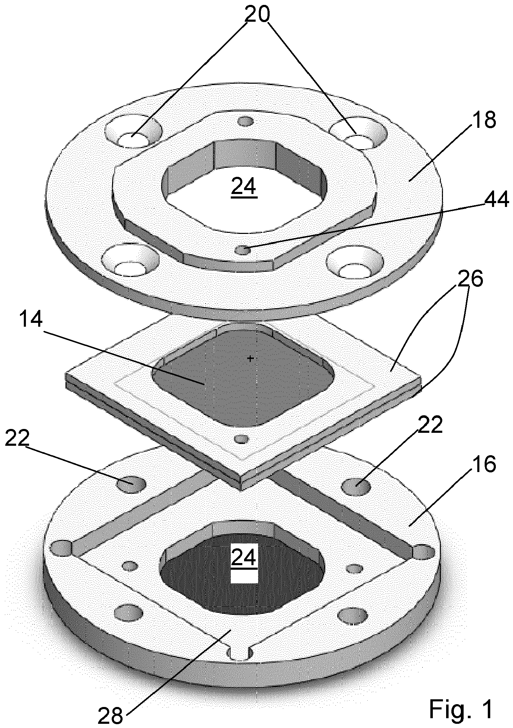

Figure 1 shows an exploded view of the different elements comprised in the device according to the invention.

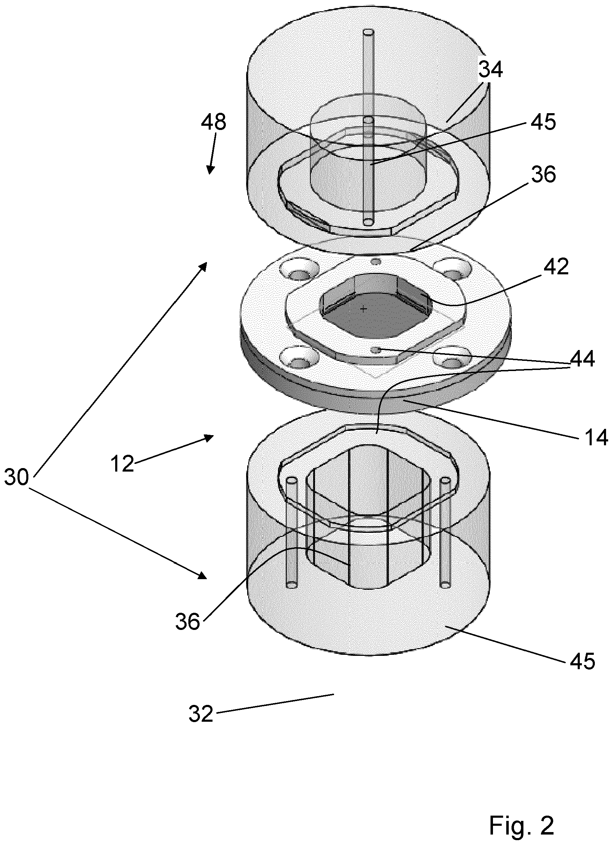

Figure 2 shows the membrane holder according to the invention with the compartments of the fluidics interface.

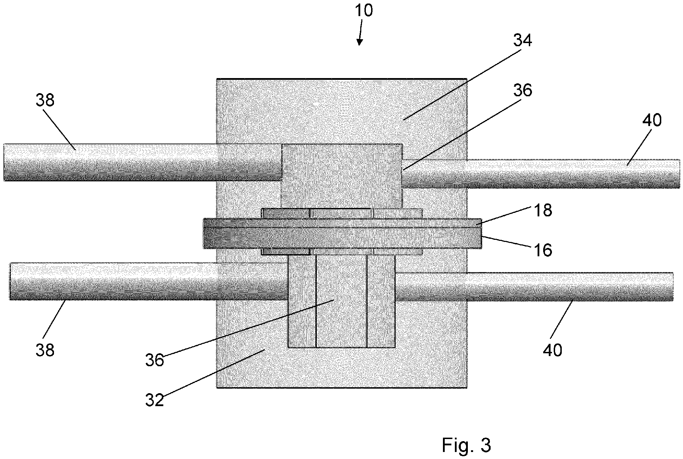

Figure 3 illustrates the device according to the invention in an assembled state.

### Detailed description

As illustrated on the drawings, the invention concerns a device 10 for being implemented both in a microwell plate and in a fluidics system.

The device 10 comprises a membrane holder 12 for a microporous membrane suited to cell culture that allows the membrane 14 to be placed in microwell plate and for cells to be cultured on the membrane 14 in the microwell plate. The membrane holder 12 is formed by a lower 16 and an upper 18 parts. These lower and upper parts are arranged to be fastened to each other and to retain the membrane 14. To this end, the lower 16 and upper 18 parts are fitted with fastening means. For example, one of said lower and upper parts comprise at least one hole 20 for cooperating with a screw (not represented) and the other of said lower and upper parts comprise at least one tapping 22 for receiving said screw, said hole 20 and said tapping 22 being arranged opposite to each other when the membrane holder 12 is assembled.

Moreover, each of said lower 16 and upper 18 parts comprises a first and a second opening 24, respectively. These openings 24 are arranged to be opposite to the membrane 14 when it is fitted in the membrane holder 12, each opening 24 being disposed on a side of the membrane 14 and allowing access to functional area of the membrane and thus making possible the permeation of the membrane 14.

Furthermore, the membrane holder 12 comprises sealing means 26, designed to be interposed between at least one of the lower 16 and upper 18 parts and the membrane 14. Preferably, the sealing means 26 are interposed, on the one hand, between the lower part 16 and the membrane 14 and, on the other hand, between the upper part 18 and the membrane 14. The sealing means 26 can be made of silicone frames or rings. Silicone frames can also comprise fastening or clipping means that allow both silicone frames to be linked together.

In order to reduce the thickness of the membrane holder 12 and to improve the positioning of the membrane 14 that will be pressed between the silicone seals, the internal sides (i.e. oriented towards the membrane) of the lower 16 and upper 18 parts may present some recesses 28 sized and shaped in such a manner that the silicone seals may take place in this recess. Preferably, the recesses 28 may present a non-circular geometry, so as to define the respective angular position of the seals in reference to the lower and upper parts. This recess 28 can be arranged either in only one of the parts 16-18 or in both.

The membrane holder 12 is sized and shaped to be introduced in and to cooperate with a microplate well. The membrane holder 12 allows to culture some cells on the membrane 14 for some time.

According to the invention, the device 10 also comprises a fluidics interface 30 defined by a lower 32 and an upper 34 compartments, designed to easily transfer the membrane 14 with cultured cells on it, in view of implementing some further experiments in a fluidics system. As it will be explained thereafter, the purpose of the invention is to transfer the membrane 14 within the membrane holder 12.

Thus, each compartment 32, 34 is formed by a 3-dimensional element that presents on one of its faces, intended to receive the membrane holder 12, a non-traversing cavity 36. The main axis of the cavity 36 may be oriented perpendicularly to the open face of the 3-dimensional element where the cavity opens onto. Each compartment 32, 34 comprises fluidics inlet 38 and outlet 40, connected with the cavity 36 and intended to be connected with fluidics channels.

The membrane holder 12 is sized and shaped to be lodged between the lower 32 and upper 34 compartments, in such a manner that the cavities 36 of said lower 32 and upper 34 compartments form, respectively with said first and second openings 24, a first and a second reaction chambers on each side of the membrane 14. The inlet 38 and the outlet 40 of the compartments 32, 34 are thus intended to feed into and to evacuate some reagents from the reaction chambers.

The lower 32 and upper 34 compartments are able to be fastened together. Some external fastening means can be implemented, like clamping devices or screws, as mentioned for the holder membrane. Both compartments 32, 34 may also be clipped together.

Similarly to the description above regarding the interaction between the sealing means 26 and the membrane holder 12, each side of the lower 32 and upper 34 compartments oriented in direction of the membrane holder 12, may present a recess 42 sized and shaped in such a manner that the membrane holder 12 or a projecting element 41 of the membrane holder may take place adjusted in this recess 42. The recess 42 may present a non circular geometry and provides a positioning system in cooperation with the membrane holder or the projecting element 41. Thus, the angular position of the membrane holder in reference to the fluidics interface is well defined.

Some additional sealing means (not represented) may also be implemented between the membrane holder 12 and each compartment 32, 34 of the fluidics device 10. The material itself of the fluidics interface 30 may allow a good watertight seal between the membrane holder 12 and the fluidics interface. For example, if the fluidics interface 30 is made in a slightly elastically deformable material, the membrane holder 12 may be inserted in force in the recesses of the compartments, ensuring a good watertight seal at the interface between the compartments and the membrane holder 12.

Thus, after the cells have been cultured on the membrane 14 for some time, the membrane holder 12 being placed in a microwell, the membrane holder 12 may then be transferred to the fluidics system, together with the cells grown on it, so that studies may be carried out on the mature cell layer.

The function of the membrane holder 12 is to allow easy handling of the membrane 14. It also provides a support for the membrane 14 in the microwell plate to ensure that the membrane is optimally positioned for cell culture and that the fragile membrane is not damaged by coming into direct contact with the microwell plate.

Different holders may be designed for different membrane types, for example, flexible polymer membranes may require a different support from porous alumina membranes. Holders may also be made for silicon chips that contain microfabricated silicon nitride microporous membranes.

In addition to its function as a mechanical support, the membrane holder 12 may also be used to protect certain structures associated with the membrane while it is in the microwell plate, and to expose these structures for use in the fluidics system. For example, microfabricated silicon chips with silicon nitride membranes may also have electrodes on the chip for electrical measurements of the cell layers formed on the membrane. In order to make a good electrical contact to these electrodes in the fluidics system, it is necessary to keep a certain part of the electrode structure (the contact pad) dry. This can be done if the holder keeps the contact pad dry in the microwell plate. Once removed from the microwell plate, the pad is exposed to the air and the holder is placed in the fluidics system, which is designed to once again protect the contact pad from the liquid cell culture medium. For example, the membrane holder 12 may comprise a passage 44 leading to the contact pad. One can here understand the importance of the positioning of the holder parts in reference to the membrane. This passage 44 can be occluded while the membrane holder 12 is used in the microwell plate. After the membrane holder 12 is lodged in the fluidics interface, the passage is not in contact with the liquid but is exposed to the air through a channel 45 arranged in the 3-dimensional element. The contact pad can therefore be connected with a measure electrode. The positioning of the fluidics interface in reference to the membrane holder is therefore also important.

The invention also concerns a method for using a membrane, consisting in disposing such membrane 14 in a membrane holder 12 as described above and performing a cell culture in a microwell plate. Then, the membrane holder 12 with the membrane and the cells grown on it, is removed from the microwell plate and disposed in a fluidics interface 30 as described above. The fluidics interface 30 can then be connected to the fluidics system in order to perform some further experiments on the cells grown on the membrane.

The examples above should not be considered as limiting. Those skilled in the art will appreciate that numerous modifications can be made thereof without departing from its spirit. The scope of the invention is to be determined by the appended claims and their equivalent.

## Claims

1. Cell culture device comprising:
- a fluidics interface (30) comprising a lower (32) and an upper (34) compartments, each compartment comprising fluidics inlet (38) and outlet (40),
- a membrane holder (12) formed by a lower (16) and an upper (18) parts, said lower and upper parts being arranged to be fastened to each other and to retain a membrane suited to cell culture, each of said lower and upper parts comprising a first and a second openings allowing possible membrane permeation,
said membrane holder (12) being sized and shaped to be introduced in and to cooperate with a microplate well,
said membrane holder (12) being sized and shaped to be lodged between the lower and upper compartments of said fluidics interface, in such a manner that said lower and upper compartments form, respectively with said first and second openings, a first and a second fluidics reaction chambers on each side of the membrane, intended to be connected with a fluidics system by said fluidics inlet (38) and outlet (40).

2. Cell culture device according to claim 1, **characterized in that** lower (16) and upper (18) parts of said membrane holder (12) comprise fastening means.

3. Cell culture device according to claim 2, **characterized in that** one of said lower (16) and upper (18) parts comprise at least one hole (20) for cooperating with a screw and the other of said lower and upper parts comprise at least one tapping (22) for receiving said screw, said hole and said tapping being arranged opposite to each other.

4. Cell culture device according to one of the preceding claims, **characterized in that** said each of said lower (32) and upper (34) compartments of the fluidics interface, are connected to a first and to a second channels, respectively defining said inlet and said outlet.

5. Cell culture device according to one of the preceding claims, **characterized in that** it comprises sealing means (26) interposed between at least one of the lower and upper parts of the membrane holder (12) and the membrane.

6. Cell culture device according to claim 5, **characterized in that** sealing means (26) are silicone seals, a first silicone seal being arranged to seal the interface between the upper side of the membrane and the upper part of the membrane holder (12) and a second silicone seal being arranged to seal the interface between the lower side of the membrane and the lower part of the membrane holder (12).

7. Cell culture device according to claim 5, **characterized in that** at least one of the upper (16) and lower (18) parts comprise a recess (28) to receive at least one of the silicone seal.

8. Cell culture device according to one of the preceding claims, **characterized in that** the upper and the lower parts of the membrane holder (12) comprise openings (24) arranged to let access to functional area of the membrane.

9. Cell culture device according to one of the preceding claims, **characterized in that** each side of the lower and upper compartments oriented in direction of the membrane holder (12), may present a recess (42) sized and shaped in such a manner that the membrane holder (12) may take place at least partially in this recess.

10. Assembly of the cell culture device (10) according to one of the preceding claims and a membrane (14), **characterized in that** said membrane is microfabricated silicon nitride microporous membrane made on a microfabricated silicon chip.

11. Assembly according to claim 10, **characterized in that** said membrane (14) comprises a chip with an electrode and **in that** said membrane holder (12) comprises a passage leading to said electrode.

12. Method for using a membrane, comprising the following steps:
- disposing such membrane in a membrane holder (12) as defined in claim 1 and performing a cell culture in a microwell plate,
- removing the microwell plate from the membrane holder (12) with the membrane and the cells grown on it,
- disposing the membrane holder (12) in a fluidics interface as defined in claim 1,
- connecting the fluidics system in order to perform experiments on the cells grown on the membrane.
